# EUROPEAN PATENT SPECIFICATION

(11) **EP 3 897 134 B1**
(45) Date of publication and mention of the grant of the patent: **08.01.2025**
(21) Application number: 18943163.8
(22) Date of filing: 14.12.2018
(51) Int. Cl.: A01N 59/12, A01P 1/00, A61K 47/10, A61P 31/04, A01N 25/22, A01N 25/04, A01N 25/30, A61K 9/00

(54) **STABLE IODINE-CONTAINING ANTIMICROBIAL TEAT DIP COMPOSITIONS**
STABILE IODHALTIGE ANTIMIKROBIELLE ZITZENBADZUSAMMENSETZUNG
COMPOSITIONS ANTIMICROBIENNES STABLES CONTENANT DE L'IODE POUR TREMPAGE DE TRAYONS

(43) Date of publication of application: 27.10.2021
(73) Proprietor: Ecolab USA Inc., St. Paul, MN 55102 (US)
(72) Inventor: GAO, Jinsen, Shanghai 200051 (CN); ZHENG, Yan, Shanghai 201204 (CN); COURT, Colin, Castle Hill, New South Wales 2159 (AU); RONEN, Shahar, East Ryde, New South Wales 2113 (AU)
(74) Representative: Godemeyer Blum Lenze Patentanwälte Partnerschaft mbB - werkpatent
(86) International application number: PCT/CN2018/121124
(87) International publication number: WO 2020/118660

(56) References cited:
- WO-A1-01/82702
- WO-A1-86/05510
- WO-A1-94/06444
- WO-A1-94/23581
- WO-A1-98/43481
- CN-A- 105 188 775
- CN-A- 107 809 908
- CN-A- 108 601 723
- GB-A- 1 516 653
- US-A- 5 368 868
- US-A- 5 529 770
- US-A- 5 776 479
- US-A- 6 042 818
- ZHENG, JUNMIN: "Pharmaceutical Polymer Materials", 31 December 2009, CHINA MEDICAL SCIENCE AND TECHNOLOGY PRESS, CN, ISBN: 978-7-5067-4021-0, article ZHENG, JUNMIN: "Passage; Pharmaceutical Polymer Materials", pages: 217 - 219, XP009529280
- LI, KAIMING ET AL: "Passage; Theories and Methods for Environmental Management of Key Water Pollution Sources in River Basins", THEORIES AND METHODS FOR ENVIRONMENTAL MANAGEMENT OF KEY WATER POLLUTION SOURCES IN RIVER BASINS, 31 December 2013 (2013-12-31), CN, pages 145 - 151, XP009529281, ISBN: 978-7-5111-1599-7
- ZHANG, HONGMEI: "Overseas Information", CHINA WASHING PRODUCTS INDUSTRY, no. 6, 31 December 2007 (2007-12-31), pages 89 - 90, XP009529282, ISSN: 1672-2701

## Description

### Technical Field

The present disclosure relates to compositions used in the treatment or prevention of environmental and contagious mastitis in mammals, such as cows, undergoing common milking operations. Specifically, embodiments of the present disclosure relate to aqueous iodine-based teat dips which include a stabilizing copolymer composition containing a mixture of poly(ethylene oxide)-poly(propylene oxide)-poly(ethylene oxide) block copolymers.

### Background

The effective management and maintenance of large dairy herds and the production of dairy products has been a major agricultural accomplishment. One of the problems in maintaining large herds is the health of the individual animals. One health problem in individual animals of dairy herds that causes significant economic problems relates to mastitis. Often, during milking, the animal's skin is irritated by automated milking machines. A typical milking machine mechanically simulates the hand milking of a cow through the use of a pulsating vacuum. For example, a vacuum pump is attached to the teat, and the vacuum is then pulsated to alternately allow the milk to fill and drain from the area of the udder and teat. Milking times may be reduced by increasing the vacuum strength, however, this practice causes irritation or damage to the teat and udder. Exposure of damaged tissue to certain microorganisms can result in an infection known as mastitis. Animals that contract mastitis must be removed from service, resulting in the loss of dairy output. As a result, a significant amount of attention has been focused on preventing the development of mastitis or treating mastitis in dairy herds.

The dairy farmer is generally faced with two different types of mastitis infections. Contagious mastitis is spread during the milking process through contact between the animal and dairy equipment that may carry a mastitis pathogen. Contagious mastitis is most easily controlled by using antimicrobial post-milking teat dips. Such antimicrobial teat dips kill the bacteria that are introduced onto the surface of the animal from the milking machines. The second type of mastitis, environmental mastitis, is caused by contamination of the animal surface by materials from the barnyard environment, fields, barn interior, etc. Such pathogens include *E. coli, Streptococcus uberis, klebsiella* and others. Such contamination occurs as the animal moves through its environment. Environmental mastitis is best treated with a barrier film that protects sensitive tissues from contamination.

Many teat dip products consist of an effective antimicrobial amount of iodine. Elemental iodine is considered a broad-spectrum germicide that is capable of rapid and complete killing of bacteria on contact. Iodine's solubility in water, however, is generally low. Therefore, aqueous iodine formulations require the presence of carrier molecules such as surfactants, glycols, or polyvinyl pyrrolidone (PVP), which solubilize the iodine through complexation. Generally referred to as iodophors, these iodine-carrier molecule complexes can consist of up to 20% iodine by weight.

Previous examples of iodine-based teat dips have utilized a variety of surfactants as carrier molecules. Among the most favored complexing agents described are nonylphenol ethoxylate ("NPE") or its derivatives and linear alcohol ethoxylates ("LAE") and their derivatives. In recent years, however, NPE has been shown to be toxic to aquatic life and also identified as endocrine disrupters. These findings have led to concerns over NPE contamination in milk consumed by humans. In fact, the European Union has placed restrictions on the presence of NPE in various compositions including teat dips. Under these regulations, the concentration of NPE in the composition must be less than 0.1 wt.%. Like NPE, linear alcohol ethoxylate (LAE) derivatives have fallen under scrutiny for their potential toxicity. Several countries have banned their use in agricultural products over these concerns.

In light of the recent revelations regarding the toxicity of NPE and the potential health risks associated with exposure to LAEs, there remains a need for the development of iodine-based teat dip compositions containing a stabilized iodine-based composition in the absence of NPE, NPE derivatives, LAE and LAE derivatives.

WO 94/06444 A1 discloses a stable germicidal aqueous composition comprising 0.1 to 1.3 wt.% of available iodine, 2 to 4.5 parts of polyethoxylated polyoxypropylene having a polyoxypropylene (POP) moiety with an average molecular weight of at least 2,600 and a polyoxyethylene (POE) content of 30 to 75% by weight per part of available iodine. Further compounds such as propylene glycol, glycerin, sorbitol as well as alcohols and glycols can be added to this.

WO 98/43481 A1 discloses stable aqueous glycerin-iodine concentrates which are diluted in water to yield germicidal iodine use solutions. The concentrates include 30 to 87% by weight of glycerin, 0.15 to 15% by weight of iodine, 0.15 to 15% by weight of iodine ions, and one or more additives such as compatible wetting agents, hydrotropes, thickening agents, additional emollients and buffering systems. In Example 3, the presence of Pluronic P105 (an EO-PO copolymer) led to an unstable formulation.

WO 86/05510 A1 discloses a low-foaming composition comprising one or two non-ionic surface active agents having a cloud point of less than 40 °C in a 1% aqueous solution, a hydrotrope such as xylene sulfonate, and a co-solvent such as a lower alcohol, propylene glycol or polyethylene glycol. The first or second surface-active agent is an ethylene oxide condensate with an alkyl or dialkyl phenol, a fatty acid alcohol or acid, or a copolymer of ethylene oxide and propylene oxide.

WO 94/23581 A1 discloses a stable aqueous antimicrobial composition comprising a polyvinyl alcohol, a polymeric thickener and an antimicrobial non-ionic iodine complex composition comprising a copolymer having an EO block and a PO block. Further ingredients, including hydrotropes, can be added to this.

WO 01/82702 A1 discloses a stable iodine-based teat dip composition comprising 5 to 100 ppm of free iodine, 70 to 90% by weight of propylene glycol, and 20 to 40% by weight of water.

US 5 776 479 A discloses a stable germicidal composition that comprises a film-forming agent selected from hydroxyethylcellulose, methyl hydroxypropylcellulose and ethyl hydroxyethylcellulose, a germicidal agent comprising non-ionic surfactant complexed iodine, i.e. iodine and an EO-PO copolymer, in particular a Pluronic surfactant, and water. Further ingredients, including glycerin, propylene glycol, sorbitol or polyethylene glycol, are added to this.

GB 1 516 653 A discloses stable iodine poloxamer antiseptic gels comprising iodine, an iodide, an EO-PO copolymer and water. The molecular weight of the PO part in the copolymer is 3,500 to 4,500, and its overall molecular weight is 11,000 to 12,000.

However, none of the aforementioned documents discloses a mixture of two copolymers in which the first EO-PO copolymer component has a number-average molecular weight of 4,500 to 10,000, and the second EO-PO copolymer component has a number-average molecular weight of 1,000 to 3,600.

### Summary

The present disclosure generally relates to iodine-based antimicrobial compositions that exhibit time and temperature stability in the absence of NPE and LAE. The antimicrobial compositions of the present disclosure are suitable for a broad range of applications, but have been found to be particularly useful for application to the teats and udders of dairy animals as udder and teat washes, and as pre-milking and post-milking sanitizing solutions.

The stable iodine-based teat dip compositions of the present disclosure comprise a poly(ethylene oxide)-poly(propylene oxide)-poly(ethylene oxide) block ("EO-PO") copolymer composition comprising a first EO-PO copolymer component and a second EO-PO copolymer component, wherein the iodine-based teat dip composition is substantially free of NPE and LAE.

The present invention relates to stable iodine-based teat dip compositions comprising a first EO-PO copolymer component and a second EO-PO copolymer component, such that the first EO-PO copolymer component has a higher number-average molecular weight than the second EO-PO copolymer component. In some embodiments, the number average molecular weight of the first EO-PO copolymer component is at least 1,000 higher than that of the second EO-PO copolymer component. In still yet other embodiments, the number-average molecular weight of the first EO-PO copolymer component is at least 2,500 higher than that of the second EO-PO copolymer component.

According to the invention, the first EO-PO copolymer component has a number average molecular weight of 4,500 to 10,000; and a stabilizing amount of a second EO-PO copolymer component having a number average molecular weight in the range from 1,000 up to 3,600.

According to the invention, the stable iodine-based compositions comprise an EO-PO copolymer composition comprising a first EO-PO copolymer component having a number-average molecular weight of 4,500 to 10,000 and a second EO-PO copolymer component having a number average molecular weight of less than 3,600; sodium xylene sulfonate, and an antimicrobially effective amount of iodine, wherein the stable iodine-based composition is essentially completely free of NPE and LAE.

In some embodiments, the stable iodine-based compositions comprise an EO-PO copolymer composition comprising a first EO-PO copolymer component having a number-average molecular weight of 5,600 and a second EO-PO copolymer component having a number-average molecular weight of 2,900, sodium xylene sulfonate, and an antimicrobially effective amount of iodine, wherein the stable iodine-based composition is substantially free of NPE and LAE.

### Selected Definitions

Unless otherwise specified, the following terms as used herein have the meanings provided below.

"Killing" as the term is used herein includes actual killing as well as inhibition or abatement of micro-organism growth.

The term "substantially free" of a particular substance means that the compositions of the instant specification contain less than 0.5 wt.% of the recited substance. When referring to "substantially free", it is intended that the substance is not intentionally added to the compositions. The term "essentially free" of a particular substance means that the compositions of the instant specification contain less than 0.1 wt.% of the recited substance. When referring to "essentially free", it is intended that the substance is not intentionally added to the compositions. The term "essentially completely free" of a particular substance means that the compositions of the instant specification contain less than 0.01 wt.% of the recited substance. When referring to "essentially completely free", it is intended that the substance is not intentionally added to the compositions. The term "completely free" of a particular substance means that the compositions of the current specification contain less than 0.001 wt.% of the cited substance. When referring to "completely free", it is intended that the substance is not intentionally added to the compositions. The use of the term "completely free" allows for trace amounts of that substance to be included in compositions because they are present in another substance in the composition. However, it is recognized that only trace or *de minimus* amounts of a substance will be allowed when the composition is said to be "completely free" of that substance.

The term "about" generally refers to a range of numbers that one of skill in the art would consider equivalent to the recited value (i.e., having the same function or result). In many instances, the terms "about" may include numbers that are rounded to the nearest significant figure.

As used herein, the singular forms "a", "an", and "the" include plural referents unless the content clearly dictates otherwise. Thus, for example, reference to a composition containing "a compound" includes a mixture of two or more compounds. As used in this specification and the appended claims, the term "or" is generally employed in its sense including "and/or" unless the content clearly dictates otherwise.

Weight percent, percent by weight, wt.-%, and the like are synonyms that refer to the concentration of a substance as the weight of that substance divided by the weight of the composition and multiplied by 100.

The terms "preferred" and "preferably" refer to embodiments of the invention that may afford certain benefits under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure.

As used herein, the term "comprising" and variations thereof do not have a limiting meaning where these terms appear in the specification and claims.

As used herein, the term "consisting essentially of" in reference to a composition refers to the listed ingredients and does not include additional ingredients that, if present, would affect the composition. The term "consisting essentially of" may also refer to a component of the composition.

As used herein, the term "NPE" refers to nonylphenol ethyoxylate and its derivatives. NPE is a class of non-ionic surfactants including the condensation products of one mole of alkyl phenol, wherein the alkyl constituent contains from 8 to 18 carbon atoms with from 3 to 50 moles of ethylene oxide. The alkyl group can for example be represented by diisobutylene, di-amyl, polymerised propylene, isoctyl, nonyl, and di-nonyl. Examples of commercial compounds of this chemistry are available on the market under the trade name IGEPAL^{®}, manufactured by Rhone-Poulenc.

As used herein, the term "LAE" refers to linear alcohol ethoxylates. LAEs are a class of nonionic surfactants that contain a hydrophobic alkyl chain attached via an ether linkage to a hydrophilic ethylene oxide (EO) chain and have the general structure R(OCH₂CH₂)ₙOH. The alkyl chain, R, can vary in length and in the degree of linearity, but is typically between 8 and 18 carbon atoms long. The EO chain can also vary in length from 1 to 40 EO units. A LAE with the structure C₉₋₁₁ EO 6.5, for example, contains a range of alkyl chain lengths of 9-11 and averages 6.5 EO units per alkyl chain.

In the interest of brevity and conciseness, any ranges of values set forth in this specification contemplate all values within the range and are to be construed as support for claims reciting any sub-ranges having endpoints which are real number values within the specified range in question. By way of a hypothetical illustrative example, a disclosure in this specification of a range of from 1 to 5 supports claims to any of the following ranges: 1-5; 1-4; 1-3; 1-2; 2-5; 2-4; 2-3; 3-5; 3-4; and 4-5.

### Detailed Description

In order to be dependable and useful to an end user, iodine-based teat dip compositions must be stable (i.e., remain homogeneous) over a wide range of temperatures. If stability is lost, and the composition separates, the utility of the composition is significantly degraded and can present a potential hazard to the user. Generally speaking, stability in this context means that a given composition must remain homogeneous after extended storage (e.g., one week or more) at temperatures as low as 4 °C (which may be experienced in cold warehouse storage areas) or as high as 50 °C (which can occur during transport in closed vehicles). Another desirable functional characteristic for iodine-based teat dip compositions designed for topical application is the ability to be spread evenly on the skin and not to drain off so rapidly as to prevent insufficient germicidal contact time. Elemental iodine is highly insoluble in aqueous solutions. Therefore, in order to use iodine in aqueous formulations elemental iodine is usually complexed with certain carrier molecules such as surfactants, glycols, or polyvinyl pyrrolidone (PVP).

The present invention relates to an aqueous iodine-based teat dip composition comprising:
(a) an EO-PO copolymer composition comprising:
   (i) a first EO-PO copolymer component having a number-average molecular weight in the range from 4,500 to 10,000; and
   (ii) a second EO-PO copolymer component having a number-average molecular weight in the range from 1,000 to 3,600;
      the first EO-PO copolymer component having a higher number-average molecular weight than the number-average molecular weight of the second EO-PO copolymer component, and a weight ratio of the first EO-PO copolymer component to the second EO-PO copolymer component being within the range of 2:8 to 8:2;
(b) a hydrotrope component, being sodium xylene sulfonate; and
(c) an antimicrobially effective amount of iodine;
wherein the composition is stable at temperatures from 4°C to 50°C and substantially free of NPE and LAE.

Surprisingly, it has been shown that stable iodine-based teat dip compositions are provided by a copolymer composition containing a mixture of poly(ethylene oxide)-poly(propylene oxide)-poly(ethylene oxide) block copolymers ("EO-PO copolymers"). The compositions of the present disclosure have unique properties which make them superior to other iodine-based teat dips. These include shelf stability and iodine stability over a large range of temperatures.

These EO-PO copolymers are generically referred to as poloxamers. Poloxamers are tri-block copolymers of the formula A-B-A or B-A-B, where A is poly(ethylene oxide) and B is poly(propylene oxide), as seen in Scheme 1.

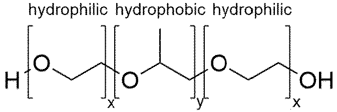

EO-PO copolymers are formed by condensing ethylene oxide with a hydrophobic base formed by the addition of propylene oxide to two hydroxyl groups of propylene glycol. Ethylene oxide is then added, to sandwich this hydrophobe between hydrophilic groups. The monomers comprising the copolymer blocks of these EO-PO copolymers are chemically dissimilar (e.g. polar and non-polar), causing them to have interesting surface activity features. For instance, EO-PO copolymers exhibit an amphiphilic character in aqueous solution on the basis of ethylene oxide solubility in water and polypropylene oxide insolubility in water. Stated another way, the EO blocks are hydrophilic, while the PO block is hydrophobic. This block segregation gives rise to useful nanostructures such as micelle formation. The size and structure of copolymer assemblies and their adsorption properties have made them useful in many applications, including drug delivery, nanoparticle synthesis and cosmetics.

Examples of commercially available EO-PO copolymers are PLURONIC^{®}, PLURONIC^{®} PE and PLURACARE^{®} copolymers manufactured by BASF Corp. and described generally in U.S. Patent No. 3,740,421 issued to Schmolka *et al.* EO-PO copolymer nomenclature varies depending on the commercial source. The naming convention for generic EO-PO copolymers includes a letter "P" with a two-digit or three-digit number, wherein the first two digits multiplied by 100 indicate the approximate molecular mass of the polypropylene oxide block, and the last digit multiplied by 10 gives the approximate percentage of polyethylene oxide block (e.g. P 407 is an EO-PO copolymer with a polypropylene oxide molecular mass of approximately 4000 g/mol and a 70% polyethylene oxide content). PLURONIC^{®} EO-PO copolymers use a different naming convention from the generic convention, with an alphabetical letter indicating the physical state (P: Paste, F: Flake, L: Liquid) of the copolymer and a two-digit or three-digit number; the first digit - or first two digits in a three-digit number - multiplied by 300 indicates the approximate molecular weight of the polypropylene oxide block, while the last digit multiplied by 10 provides approximate percentage of polyethylene oxide block (e.g. L61 indicates a EO-PO copolymer that is liquid with an approximate molecular mass of polypropylene oxide block of 1800 g/mol and approximately 10% polyethylene oxide content ). PLURONIC^{®} PE copolymers are designated by a letter and a four or five-digit number; the first digit is guide to the approximate molar mass of the polypropylene oxide block on a scale of 1-10. The second, or third, digit divided by 10 provides the percentage of polyethylene oxide. For instance, PLURONIC^{®} PE3100 has a polypropylene oxide block with an approximate molar mass of 850 g/mol and contains approximately 10% polyethylene oxide, whereas PLURONIC^{®} PE10100 has a polypropylene oxide block with an approximate molar mass of 3250 g/mol and contains approximately 40% polyethylene oxide. PLURACARE^{®} copolymers are designated by a letter, indicating the physical state of the copolymer and a three digit number. For example, PLURACARE^{®} F127 is a flake copolymer where the first two digits multiplied by 300 indicates the approximate molar mass of the polypropylene oxide block and the last digit, when multiplied by 10 indicates the approximate polyethylene oxide content of the copolymer.

Most commercial sources of EO-PO copolymers provide information regarding the number average molecular weight of the entire copolymer. The number average molecular weight can be determined by various methodologies such as fractionation, ultracentrifugation analysis, as well as, the mathematical analysis as described in FLORY, P. J. J. Am. Chem. Soc. 62, pages 1501 to 1504. In other instances, only the molar mass of the hydrophobe or central polypropylene oxide block is provided. For simplification, the molecular weights of the EO-PO copolymers described in the present disclosure are their number average molecular mass.

Examples of EO-PO copolymers preferred for the embodiments of the present disclosure include but are not limited to the following EO-PO copolymers available from BASF: PE3100, PE6200, PE6400, PE9400, PE10100, P104, P105, F127, F108. These EO-PO copolymers are of the A-B-A type shown in Scheme 1 and are available in a broad range of number-average molecular weights and EO/PO ratios.

Without wishing to be bound by theory, it is believed that the EO-PO copolymer composition forms a stable iodine-based teat dip composition by creating a physical barrier or micelle which separates the iodine particles, allowing them to stay in solution. The hydrophobic polypropylene oxide block adsorbs on the iodine particles, while the long hydrophilic polyethylene oxide block tail is orientated towards the water phase. Lengthening the hydrophilic tail causes greater extension of the shear plane and results in improved particle protection. Furthermore, a long propropylene oxide chain can provide multiple points of attachment to the particle, making desorption less likely than for conventional nonionic surfactant (which have only a single hydrophobe). The twin polyethylene oxide hydrophiles offer extra stabilization for a lower surfactant concentration and longer polyethylene oxide chains extend the shear plane further than other nonionic surfactants thus improving dispersion stability.

Preferred EO-PO copolymers for the stable iodine-based teat dip compositions of the present disclosure are chosen such that the polyethylene oxide block constituent comprises from 10 wt.% to 90 wt.%, from 20 wt.% to 70 wt.%, from 30 wt.% to 50 wt.%, from 40 wt.% to 60 wt.%, or from 50 wt.% to 80 wt.% of the total copolymer.

The present invention relates to a stable iodine-based teat dip composition comprising an EO-PO copolymer composition comprising a first EO-PO copolymer component and a second EO-PO copolymer component, wherein the first EO-PO copolymer component has a number average molecular weight that is higher than that of the second component. In some embodiments, the number average molecular weight of the first EO-PO copolymer component is at least 1000, at least 1500, at least 2,000 or at least 5,000 higher than the number-average molecular weight of the second EO-PO copolymer component.

According to the present invention, the first EO-PO copolymer component has a number average molecular weight from 4,500 to 10,000, preferably from 5,000 to 7,000, or most preferably from 5,900 to 6,500.

According to the present invention, the second EO-PO copolymer component has a number-average molecular weight from from 1,000 to 3,600, preferably from 1,000 to 2,900.

According to the present invention, the stable iodine-based composition has a weight ratio between the first EO-PO copolymer component and the second EO-PO copolymer component in the range from from 2:8 to 8:2, preferably from 3:2 to 2:3, more preferably from 0.5:1 to 1:0.5, or most preferably from 0.01:1 to 1:0.01. In some embodiments, the weight ratio between the first EO-PO copolymer component and the second EO-PO copolymer component is 1:1.

In some embodiments, the first EO-PO copolymer component is present in the range of 0.1 wt.% to 10 wt.%, 1.0 wt.% to 8 wt.%, 2 wt.% to 6 wt.%, 3 wt.% to 5 wt.%, or 4 wt.% of the total composition. In some embodiments, the second EO-PO copolymer component is present in the range of 0.1 wt.% to 10 wt.%, 1.0 wt.% to 8 wt.%, 2 wt.% to 6 wt.%, 3 wt.% to 5 wt.%, or 4 wt.% of the total composition.

In some embodiments, the total amount of EO-PO copolymer present in the iodine-based teat dip composition is in the range from 1.0 wt.% to 20 wt.%. In other embodiments, the total EO-PO copolymer present in the composition is in the range from 1.0 wt.% to 9.0 wt.%, from 1.0 wt.% to 4.0wt.%, from 8.0 wt.% to 20 wt.%, from 4.0 wt.% to 14.0 wt.%, from 5.0 wt.% to 8.0 wt.%, or from 0.5 wt.% to 2.0 wt.%. These ranges can refer to a ready-to-use composition or a concentrate, depending on the desired properties of the final teat dip composition.

In addition to the EO-PO copolymer components, the iodine-based teat dip compositions are completely free, essentially completely free or substantially free from NPE or nonionic surfactants that include the condensation products of one mole of alkyl phenol, wherein the alkyl constituent contains from 8 to 18 carbon atoms with from 3 to 50 moles of ethylene oxide. Examples of commercial compounds of this chemistry are available on the market under the trade name IGEPAL^{®} manufactured by Rhone-Poulenc and TRITON^{®} manufactured by Dow. The compositions of the present disclosure are in particular substantially free of nonyl phenol ethoxylate 12 mole (NPE 12) or IGEPAL^{®} CO-720 available from Rhone-Poulenc. The compositions contain no more than 0.1 wt.% of NPE, no more than 0.01 wt.% of NPE, or no more than 0.001 wt. % NPE of the total ready to use composition.

In addition to the EO-PO copolymer components, the iodine-based teat dip compositions are completely free, essentially completely free, or substantially free from LAE, where LAE is a class of nonionic surfactants that contain a hydrophobic alkyl chain attached via an ether linkage to a hydrophilic ethylene oxide (EO) chain and have the general structure R(OCH₂CH₂)ₙOH. The compositions contain no more than 0.1 wt. % of LAE, no more than 0.01 wt. % of LAE, or no more than 0.001 wt. % LAE of the total ready to use composition.

### Iodine Compounds

In some embodiments of the present disclosure, the iodine can be provided as an activated iodine premix, for example NaOH/I₂ or NaI/I₂. The iodine premix can be formulated by adding deionized water, iodine and an iodide constituent to a reactor with adequate mixing. Generally, the iodide constituent can be any alkaline earth metal-iodine salt such as sodium iodide or potassium iodide. An NaI/I₂ premix at a concentration of 1.8% of the total weight of the use solution provides 1% titratable I₂ in the use solution. In other embodiments, the iodine is provided by adding deionized water, iodine and sodium hydroxide to a reactor with adequate mixing. An NaOH/I₂ premix at a concentration of 1.8% of the total weight of the use solution provides 1% of titratable I₂ in the use solution. The activated iodine premix is added to the EO-PO copolymer composition. Preferred embodiments provide a titratable iodine concentration from 0.05% to 5 wt.%, from 1.0 to 3.0 wt.%, from 1.5 to 2.5 wt.%, from 2.0 wt.% to 2.5 wt.%, or from 0.3 wt.% to 1.0 wt.%. More preferably, the iodine is present in the iodine-based teat dip compositions at 0.1 wt.% to 2.5 wt.% These ranges can refer to a ready-to-use composition or a concentrate, depending on the desired properties of the final teat dip composition.

### Hydrotropes

According to the present invention, solubilizing agents called hydrotropes or couplers are added to the iodine-based teat dip compositions. Hydrotropes such as monofunctional and polyfunctional alcohols may be used to maintain physical single-phase integrity and storage stability. These contain from 1 to 6 carbon atoms and from 1 to 6 hydroxy groups. Examples include ethanol, isopropanol, n-propanol, 1,2-propanediol, 1,2-butanediol, 2-methyl-2,4-pentanediol, mannitol and glucose. Other examples include the higher glycols, polyglycols, polyoxides, glycol ethers and propylene glycol ethers. Additional hydrotropes include the free acids and alkali metal salts of sulfonated alkylaryls such as toluene, xylene, cumene and phenol or phenol ether or diphenyl ether sulfonates; alkyl and dialkyl naphthalene sulfonates and alkoxylated derivatives. Other examples of hydrotropes are xylene sulfonate, 1-octane sulfonate and 1,2-octane disulfonate.

The hydrotrope added to the iodine-based teat dip compositions is sodium xylene sulfonate ("SXS"). SXS is found in personal care products because of its ability to serve as a claritant or wetting agent that helps a formula spread more easily. The hydrotrope provides high temperature stability and cloud point control. Generally, the hydrotrope component may be present from 0.01 wt.% to 10 wt.%, from 0.1 wt.% to 10 wt.%, from 1.0 wt.% to 10 wt.%, from 4.0 wt.% to 10 wt.%, from 2 wt.% to 8 wt.%, or from 1.5 wt.% to 4.0 wt.% of the composition. These ranges can refer to a ready-to-use composition or a concentrate composition, depending on the desired properties of the final teat dip composition.

### Surfactants/Wetting Agents

Although EO-PO copolymers exhibit non-ionic surfactant properties, the presence of additional wetting agents or surfactants have been found to be beneficial for the iodine teat dip compositions of the present disclosure. Wetting agents such as surfactants may be included, to formulate the disclosed compositions for an intended environment of use. Typical wetting agents are used to wet the surface of application, reduce surface tension of the surface of application so that the product can penetrate easily on the surface, and to remove unwanted soil. The wetting agents or surfactants of the formulation increase the overall detergency of the formula, solubilize or emulsify some of the organic ingredients that otherwise would not dissolve or emulsify, and facilitate penetration of active ingredients deep onto the intended surface of application, such as animal skin.

Suitable surfactants may include but are not limited to anionic, cationic, nonionic, zwitterionic and amphoteric surfactants. Suitable anionic surfactants can be chosen from alkyl sulfonic acid, alkyl sulfonate salt, alkyl sulfate salt, linear alkylbenzene sulfonic acid, a linear alkylbenzene sulfonate salt, alkyl α-sulfomethyl ester, alkyl α-olefin sulfonate salt, alcohol ether sulfate salt, alkyl sulfate salt, alkylsulfo-succinate salt, a dialkylsulfosuccinate salt, and their alkali metal, alkaline earth metal, amine and ammonium salts thereof. Specific examples are linear C₁₀-C₁₆ alkylbenzene sulfonic acid, linear C₁₀-C₁₆ alkylbenzene sulfonate or alkali metal, alkaline earth metal, amine, alkanol amine and ammonium salts thereof, e.g., sodium xylene sulfonate, sodium dodecylbenzene sulfonate, sodium octane sulfonate, sodium lauryl sulfate, sodium C₁₄-C₁₆ α-olefin sulfonate, C₁₂-C₁₈ sodium methyl α-sulfomethyl ester and C₁₂-C₁₈ disodium methyl α-sulfo fatty acid salt. Suitable nonionic surfactants can be chosen from alkyl polyglucoside, alkyl ethoxylated alcohol, alkyl propoxylated alcohol, ethoxylated propoxylated alcohol, sorbitan, sorbitan ester and alkanol amide. Specific examples include C₈-C₁₆ alkyl polyglucoside with a degree of polymerization ranging from 1 to 3, e.g., C₈-C₁₀ alkyl polyglucoside with a degree of polymerization of 1.5 (Glucopon^{®} 200), C₈-C₁₆ alkyl polyglucoside with a degree of polymerization of 1.45 (Glucopon^{®} 425), C₁₂-C₁₆ alkyl polyglucoside with a degree of polymerization of 1.6 (Glucopon^{®} 625). Other useful non-ionic surfactants include condensation products of one mole of saturated or unsaturated, straight-chain or branched-chain carboxylic acid having from 8 to 18 carbon atoms with from 6 to 50 moles of ethylene oxide. The acid moiety can consist of mixtures of acids in the above delineated carbon atom range or can consist of an acid having a specific number of carbon atoms within the range. Examples of commercial compounds of this chemistry are available on the market under the trade name NOPALCOL^{®} manufactured by Henkel Corporation and LIPOPEG^{®} manufactured by Lipo Chemicals, Inc. In addition to ethoxylated carboxylic acids, commonly called polyethylene glycol esters, other alkanoic acid esters formed by reaction with glycerides, glycerin, and polyhydric (saccharide or sorbitan/sorbitol) alcohols can be used in the composition. All of these ester moieties have one or more reactive hydrogen sites on their molecule which can undergo further acylation or ethylene oxide (alkoxide) addition to control the hydrophilicity of these substances.

Amphoteric surfactants can be chosen from alkyl betaines, alkylamido betaines, alkylamidoalkyl betaines and alkyl amphoacetates. Suitable betaines include cocoamidopropyl betaine, and suitable amphoacetates include sodium cocoamphoacetate, sodium lauroamphoacetate and sodium cocoamphodiacetate.

### Thickening Agents

A composition of the present disclosure may optionally contain one or more rheology modifiers, to enhance viscosity, or thicken the composition to facilitate adherence of a dip to the teat. Adherence enables the composition to remain in contact with transient and resident pathogenic bacteria for longer periods of time, promoting microbiological efficacy and resisting waste because of excessive dripping. The rheology modifier may be a film former or act cooperatively with a film-forming agent to form a barrier that provides additional protection. An important aspect of this thickening is that the iodine teat dip composition is fluid enough for pouring or dipping but still has sufficient thixotropy or viscosity to resist rapid draining or running off from the teat or udder.

A variety of organic and inorganic agents can increase the viscosity, apparent viscosity, or shear-dependent viscosity (thixotropy) of water. Preferred aqueous thickening agents which are more useful in the compositions of the present disclosure are those which are non-Newtonian (psuedoplastic) that tend not to develop rigid intermolecular interactions. Inorganic thickeners are generally compounds such as colloidal magnesium aluminum silicate (VEEGUM^{®}), colloidal clays (Bentonites), or silicas (CAB-O-SILS^{®}) which have been fumed or precipitated to create particles with large surface -to -size ratios. Suitable natural hydrogel thickeners are primarily: vegetable -derived exudates, e.g., tragacanth, karaya, and acacia gums; extractives, e.g., caragheenan, locust bean gum, guar gum and pectin; or pure -culture fermentation products such as xanthan gum. Chemically, all of these materials are salts of complex anionic polysaccharides. Synthetic natural-based thickeners having application are cellulosic derivatives wherein the free hydroxyl groups on the linear anhydro-glucose polymers have been etherified or esterified to give a family of substances which dissolve in water and give viscous solutions. This group of materials includes the alkyl and hydroxylalkycelluloses, specifically methylcellulose, hydroxyethylmethylcellulose, hydroxypropylmethylcellulose, hydroxybutylmethylcellulose, hydroxyethylcellulose, ethylhydroxyethylcellulose, hydroxypropylcellulose, and carboxymethylcellulose. Synthetic petroleum-based water soluble polymers are prepared by direct polymerization of suitable monomers of which polyvinylpyrrolidone, polyvinylmethylether, polyacrylic acid and polymethacrylic acid, polyacrylamide, polyethylene oxide, and polyethyleneimine are representative. A preferred rheology modifier is xanthan gum, for example KELZAN^{™}-T, manufactured by Kelco Biopolymer. This rheology modifier is particularly advantageous in that it is a pseudoelastic composition having non--thixotropic properties.

The ready-to-use teat dip compositions may benefit from a preferred dynamic viscosity ranging from 50 cP to 4000 cP, from 100 cP to 3000 cP, from 200 cP to 2000 cP, 200 cP to 1000 cP, 300 cP to 800 cP, or from 300 cP to 700 cP (Brookfield Spindle #2 at 100 rpm at 23 °C) at the time of the composition is to be applied to the skin of an animal. In preferred embodiments, the ready-to-use iodine-based teat dip compositions have a dynamic viscosity of less than 500 cP. Generally, thickeners may be present from 0.01 wt.% to 10 wt.%, from 0.1 wt.% to 10 wt.%, from 1.0 wt.% to 10 wt.%, from 0.5 wt.% to 9 wt.%, from 1.0 wt.% to 8.0 wt.%, and from 5.0 wt.% to 7.0 wt.% of the composition. These ranges can refer to a ready-to-use composition or a concentrate composition, depending on the desired properties of the final teat dip composition.

### Emollients/Skin Conditioning Agents

The disclosed compositions may optionally contain an emollient or skin conditioning agent to assist in forming a protective coating on the skin to retain moisture. For example, skin conditioning agents may include moisturizers, such as glycerin, sorbitol, propylene glycol, Laneth-5 to 100, lanolin, lanolin alcohol, lanolin ethoxylate/alkoxylated lanolin, allantoin, D-panthenol, polyethylene glycol (PEG) 200-10,000, polyethylene glycol esters, monoglyceryl fatty alkanoate, acyl lactylates, polyquaternium-7, glycerol cocoate/laurate, PEG-7 glycerol cocoate, stearic acid, hydrolyzed silk peptide, silk protein, hydroxypropyl trimonium chloride, alkyl poly glucoside/glyceryl laurate, B₅ provitamin, polysorbate 80 (Tween 80), shea butter and cocoa butter; sunscreen agents, such as titanium dioxide, zinc oxide, octyl methoxycinnamate (OMC), 4-methylbenzylidene camphor (4-MBC), avobenzone, oxybenzone and homosalate; and itch-relief or numbing agents, such as aloe vera, calamine, mint, menthol, camphor, antihistamines, corticosteroids, benzocaine and paroxamine HCl.

Some emollients also perform as thickening agents. An important aspect of the teat dip formula is that it is fluid enough for pouring or dipping but still has sufficient thixotropy or viscosity to resist rapid draining or running off from the teat or udder. Generally, emollients may be present from 0.01 wt.% to 10 wt.%, from 0.1 wt.% to 10 wt.%, from 1.0 wt.% to 10 wt.%, from 0.5 wt.% to 9 wt.%, from 1 wt.% to 8 wt.% and from 5 wt.% to 7 wt.% of the composition. These ranges can refer to a ready-to-use composition or a concentrate composition, depending on the desired properties of the final teat dip composition.

### Chelates

In some embodiments, the composition may optionally include a chelating agent. For example, for cost saving purposes, it may be desirable to provide a concentrate which may be diluted by the distributor or at the farm. It is cheaper to store and transport a concentrate as compared to a ready to use solution which is heavier and takes up more volume. However, it has been found that the effectiveness of a concentrated teat dip may suffer when the concentrate is diluted with poor quality water. The addition of a chelate has been found to improve the efficiency of compositions adversely affected if the dilution water used to prepare the use solution is less than satisfactory. A chelate is a substance whose molecules can form one or more bonds with a metal ion. In particular, water often contains metal ions, such as calcium ions, that might react with anionic components (e.g. surfactants, acids, etc.) present within the teat dip composition. Without being limited to theory, it is believed that a chelate can form a complex with such metal ions so that the remaining anionic components are capable of fulfilling their desired function. Moreover, in some instances, it is also believed that the chelate can improve the ability of the teat dip composition to inhibit the growth of gram negative and/or gram positive bacteria.

Some examples of chelates that may be used in the teat dip composition include, but are not limited to, hydroxyethylidene-1,1-diphosphonic acid (HEDP), ethylenediamines, ethylenediaminetetraacetic acid (EDTA) and/or salts thereof, citric acids and/or salts thereof, glucuronic acids and/or salts thereof, polyphosphates, organophosphates, dimercaprols and the like. The amount of the chelate utilized in the teat dip composition can generally vary depending on the relative amounts of the other components present within the formulation. Typically, when utilized, the chelate is present in the formulation in an amount between 0.01 wt.% to 5 wt.%, 0.25 wt.% to 4 wt.%, 0.3 wt.% to 3 wt.% or 0.5 wt.% to 2 wt.%. These ranges can refer to a ready-to-use composition or a concentrate composition, depending on the desired properties of the final teat dip composition.

### pH Modifiers

In some embodiments, the compositions may optionally include a pH modifier. Maintenance of the pH of compositions is preferred, in order to minimize undesirable chemical changes which may inhibit the microbiological efficacy of the antimicrobial components or cause a toxic or irritating effect upon the teat. Any compatible organic or inorganic material or mixture of materials which has the desired effect of maintaining the composition pH within prescribed ranges can be utilized as the buffering agent or system. Factors which may cause undesirable pH shifts include the presence of naturally occurring chemicals brought into the composition, after application onto the teat, by skin exudations, milk or environmental soils; and, pH drifting which sometimes accompanies chemical equilibria established within compositions, as ingredients are changed or concentrations varied, for example concentration changes which can occur as a teat dip dries on the teat. In addition, in some embodiments, the compositions are concentrates which may be diluted by the distributor or at the farm to form an aqueous solution. Even if neutral, softened, distilled, or deionized water is used, adjustment of the pH of the teat dip to the desired range and stabilization of the adjusted pH with a pH modifier may be necessary.

The amount of pH modifier employed in a particular formulation is chosen on the basis of pH stability characteristics determined over a period of time. In general, the pH of iodine-containing teat dips can vary from a low pH of 2.5 to a maximum of approximately 10.5 depending primarily upon the choice of antimicrobial agent being incorporated in the composition. Therefore, the pH modifier or system is chosen accordingly. The pH range of the disclosed compositions is preferably from2.0 to 10.0, from 3.0 to 7.0, from 5.0 to 7.0, from 4.5 to 6.5, from 4.0 to 6.0 or from 3.5 to 5.5.

Some examples of basic pH modifiers include, but are not limited to: ammonia; mono-, di- and tri-alkyl amines; mono-, di- and tri-alkanolamines; alkali metal and alkaline earth metal hydroxides; alkali metal and alkaline earth metal silicates; and mixtures thereof. Specific examples of basic pH modifiers are: ammonia; sodium, potassium and lithium hydroxide; sodium, potassium and lithium meta silicates; monoethanolamine; triethylamine; isopropanolamine; diethanolamine; and triethanolamine.

Some examples of acidic pH modifiers include, but are not limited to, mineral acids, carboxylic acids and polymeric acids. Useful weak inorganic acids include phosphoric acid and sulfamic acid. Useful weak organic acids include acetic acid, hydroxyacetic acid, citric acid, tartaric acid, lactic acid, glycolic acid, adipic acid, succinic acid, propionic acid, malic acid, alkane sulfonic acids, cycloalkane sulfonic acids, etc. Specific examples of suitable mineral acids are hydrochloric acid, nitric acid, and sulfuric acid. Specific examples of suitable carboxylic acids are maleic acid, malic acid, succinic acid, glutaric acid, benzoic acid, malonic acid, salicylic acid, gluconic acid and mixtures thereof. Specific examples of suitable polymeric acids include straight-chain poly(acrylic) acid and its copolymers (e.g., maleic-acrylic, sulfonic-acrylic, and styrene-acrylic copolymers), cross-linked polyacrylic acids having a molecular weight of less than 250,000, poly(methacrylic) acid, and naturally occurring polymeric acids such as carageenic acid, carboxymethyl cellulose and alginic acid. Mixtures of organic and inorganic acids can also be used. One typical and preferred buffer system is citric acid and its alkali metal salt.

In some embodiments of the present invention, the pH modifier is sodium hydroxide. When utilized, the amount of the pH modifier can be present in any effective amount needed to achieve the desired pH level. For example, in some embodiments, the pH modifier is present in the formulation in an amount from 0.001 wt.% to 5.0 wt.%, from 0.001 wt.% to 5.0 wt.%, or from 0.1 wt.% to 0.25 wt.% of the teat dip composition. In particular embodiments, the pH modifier is present in an amount from 0.001 wt.% to 0.2 wt.% of the teat dip composition. These ranges can refer to a ready-to-use composition or a concentrate composition, depending on the desired properties of the final teat dip composition.

### Additional Components

The compositions may also optionally include: medicaments, for example sunscreens such as para-amino benzoic acid, zinc oxide, avobenzone, and oxybenzone; healing agents such as allantoin or urea, to provide curative action and stimulate the formation of new tissue; preservatives such as methyl paraben, propyl paraben, sorbic and benzoic acids or salts thereof, to retard bacterial growth and prolong shelf life; antioxidants such as BHT (butylated hydroxytoluene), BHA (butylated hydroxyanisole), TBHQ (tert-butylhydroquinone) or propyl gallate, to retard oxidative or hydrolytic degradation; sequestering agents such as aminopolyacetates, polyphosphonates, aminopolyphosphonates, polycarboxylates and condensed phosphates; dispersants or suspending agents having polyelectrolytic character such as polyacrylate and similar polycarboxylates having a homopolymeric or copolymeric structure; and manufacturing processing agents, for example defoaming additives employed to facilitate blending and mixing.

### Methods of Making and Using

The disclosed compositions can be in the form of a liquid or solid including, but not limited to, emulsions, micro-emulsions, thickened gels, liquids or powders.

The stable iodine teat dip compositions of the present disclosure may be concentrated or ready-to-use compositions. In general, a concentrate refers to a composition that is intended to be diluted with water or another diluent to provide a use solution that contacts an object to provide the desired effect. Such concentrates offer the advantages of minimizing manufacturing, packaging, shipping, and storage costs. Generally speaking, concentrates usually contain from 15% by weight of available iodine and are usually formulated to provide 5, 10 or 15 times the original concentrate volume, after dilution with an appropriate amount of diluent. Alternatively, the ready-to-use solutions can be made directly without the preparation of concentrates, i.e. diluting the concentrate to form a use solution. The compositions can be formulated to provide a ready-to-use composition with a higher concentration of materials in order to provide a high level of beneficial properties such as antimicrobial activity, vertical cling, skin health or softening, etc. Conversely, compositions can be formulated to provide a ready-to-use composition with diluted concentration ranges to provide milder benefits such as mild antimicrobial properties, less viscosity (e.g. in the case of an udder wash or dip) or overall mildness to avoid causing skin irritation. For these reasons, the concentration ranges provided in this disclosure can encompass both a concentrate composition and a ready-to-use composition, depending on the desired properties and benefits of the teat dip composition.

The iodine teat dip compositions can be applied to the area to be treated in a variety of ways. The compositions may be applied onto a surface as a spray or foam or by dipping, i.e. submerging, or wiping the surface in the use solution. The compositions of the present disclosure, when applied to the teats of agricultural animals, dry to form a continuous barrier film on the animal's udder and over the teat of the animal which has high adhesion characteristics and which will not substantially crack, fall off, rub off or wash off in the field. The composition can nonetheless still be readily removed before milking, by rinsing or scrubbing with water or other typical pre-milking udder preparation.

The present disclosure may be better understood with reference to the following examples. These examples are intended to be representative of specific embodiments of the disclosure and are not intended to limit the scope of the disclosure.

### Examples

TABLE 1 shows the weight ranges of ingredients useful for the compositions of the present disclosure.

**TABLE 1**

| Component (% w/w) | Formulation 1 | Formulation 2 |
|---|---|---|
| Water | 70-80 | 70-80 |
| Total EO-PO copolymer | 0.25-10 | 1-6 |
| Iodine | 0.05-3 | 0.1-2.5 |
| Hydrotrope | 0.1-10 | 5-10 |
| Chelate (optional) | 0-2 | 0.01-0.5 |
| Thickener (optional) | 1-5 | 2-4.5 |
| Emollient | 0-12 | 2-12 |
| pH modifiers | 0.05-3 | 0.01-2 |

A representative formulation is shown in Table 2

**Table 2**

| Component | Wt.% |
|---|---|
| Water | 73 |
| First EO-PO copolymer component | 2.5 |
| Second EO-PO copolymer component | 2.5 |
| Iodine | 2.3 |
| Hydrotrope | 10 |
| Chelate (optional) | 0.1 |
| Thickener (optional) | 0-0.5 |
| Emollient | 0-12 |
| Sodium hydroxide | 0.75 |
| Phosphoric Acid | 1.7 |

The compositions were prepared as follows: Course iodine was dissolved in an alkali solution over the course of an hour. A pre-mixed solution of the first EO-PO copolymer and the second EO-PO copolymer components was added to the stirred iodine solution, followed by a thickener and emollient. The composition was stirred for one hour at room temperature. The hydrotrope was then added to the stirred solution, followed by the addition of phosphoric acid and a chelating agent. The pH of the composition was then adjusted accordingly using an alkali silicate.

The effect of EO-PO copolymers on the stability of the iodine-based teat dip composition was determined by preparing various compositions according to Table 1, wherein the EO-PO copolymers were selected based on molecular weight and wt.% of EO. The stability of each of these formulations was determined at 4 °C, room temperature, 40 °C and 50 °C by visual inspection, where cloudiness in the solution was determined to be unstable. The results are shown in Table 3.

**TABLE 3**

| **Trade name** | **Stability(appearance)** | | | | **Number-Average Molecular Weight** | **EO %** |
|---|---|---|---|---|---|---|
| | **4 °C** | **25 °C** | **40 °C** | **50 °C** | | |
| PE3100 | unstable | unstable | unstable | unstable | 1000 | 10 |
| PE6200 | unstable | unstable | unstable | unstable | 2450 | 20 |
| PE6400 | unstable | unstable | unstable | unstable | 2900 | 40 |
| PE9400 | unstable | stable | unstable | unstable | 4600 | 40 |
| P10100 | unstable | unstable | unstable | unstable | 3500 | 10 |
| P104 | unstable | unstable | unstable | unstable | 5900 | 40 |
| P105 | unstable | unstable | unstable | unstable | 6500 | 50 |
| PF127* | unstable | unstable | unstable | unstable | 126000 | 70 |
| PF108* | unstable | unstable | unstable | unstable | 146000 | 80 |

| | | | | | | |
|---|---|---|---|---|---|---|
| * = reference examples | | | | | | |

The data in Table 3 show that compositions containing only a single EO-PO copolymer are not stable throughout the desired temperature range of 4 °C to 50 °C. Compositions containing a single EO-PO copolymer with number-average molecular weights within the range of 1,000 to 3,600 failed to provide stable compositions at 4 °C and above 50 °C, while compositions containing a single EO-PO copolymer with average molecular weights within the range of 4,600 to 6,500 produced compositions that gelled during mixing. Further compositions containing a single EO-PO copolymer with average molecular weights greater than 10,000 failed to produce stable iodine-based compositions.

The effect of a combination of a first EO-PO copolymer component and a second EO-PO copolymer component on the stability of the iodine-based teat dip composition was determined by preparing various compositions according to Table 1. The stability of each of these formulation was determined at 4 °C, room temperature, 40 °C and 50 °C as described above. The results are shown in Table 4.

**TABLE 4**

| **Trade name** | **Stability(appearance)** | | | | **Number Average Molecular Weight** |
|---|---|---|---|---|---|
| | **4 °C** | **25 °C** | **40 °C** | **50 °C** | |
| P104 + SXS* | unstable | unstable | unstable | unstable | 5900 |
| P104 + PE6200* | Stable, cloudy appearance | stable | stable | unstable | 5900 + 2450 |
| P104+PE3100+SXS | stable | stable | stable | stable | 5900+1000 |
| P104 + PE6200 + SXS | stable | stable | stable | stable | 5900 + 2450 |
| P104 + PE6400* | Stable, cloudy appearance | stable | stable | unstable | 5900 + 2900 |
| P104 + PE6400 + SXS | stable | stable | stable | stable | 5900 + 2900 |
| PE6400 + SXS* | unstable | stable | stable | stable | 2900 |
| P10100+PE6400+SXS* | unstable | unstable | unstable | unstable | 3500+2900 |
| F108+PE6400+SXS* | unstable | unstable | unstable | unstable | 14600+2900 |
| F127+PE6400+SXS* | unstable | stable | stable | stable | 12600+2900 |

| | | | | | |
|---|---|---|---|---|---|
| * = reference examples | | | | | |

The data in Table 4 show that a stable iodine-based composition is formed when an anionic surfactant is used in combination with an EO-PO copolymer composition comprising a first EO-PO copolymer component with a number average molecular weight greater than 3,600 and a stabilizing second EO-PO copolymer with a number average molecular weight below 3,600. Compositions containing the hydrotrope SXS and the copolymer composition with a first EO-PO copolymer component with a number average molecular weight of 5,900 and a second EO-PO copolymer component with a number average molecular weight of 2,490 to 2,900 produced stable iodine-based compositions across the entire tested temperature range. Thus, the combination of a high number-average molecular weight EO-PO copolymer with a lower number-average molecular weight EO-PO copolymer provides stable iodine-based teat dip compositions with an antimicrobially effective amount of iodine after prolonged storage at high temperatures.

Finally, the effect of pH on the compositions of the present disclosure was also tested. Compositions with a pH of less than 3.5 provided stable iodine-based teat dip compositions at prolonged storage at 50 °C.

## Claims

1. An aqueous iodine-based teat dip composition comprising:
(a) an EO-PO copolymer composition comprising:
(i) a first EO-PO copolymer component having a number average molecular weight in the range from 4,500 to 10,000; and
(ii) a second EO-PO copolymer component having a number average
molecular weight in the range from 1,000 to 3,600;
the first EO-PO copolymer component having a higher number average molecular weight than the number average molecular weight of the second EO-PO copolymer component; and a weight ratio of the first EO-PO copolymer component to the second EO-PO copolymer component being within the range of 2:8 to 8:2;
(b) a hydrotrope component, being sodium xylene sulfonate; and
(c) an antimicrobially effective amount of iodine;
wherein the composition is stable at temperatures from 4°C to 50°C and substantially free of NPE and LAE.

2. The composition of claim 1, wherein the first EO-PO copolymer component has a number-average molecular weight from 5,900 to 6,500.

3. The composition of claim 1, wherein the second EO-PO copolymer component has a number-average molecular weight from 1,000 to 2,900.

4. The composition of claim 1, wherein the antimicrobial iodine is selected from the group consisting of iodine, sodium iodide, potassium iodide, NaOH/I₂ and mixtures thereof.

5. The composition of claim 1, wherein the concentration of the hydrotrope component in the composition is from 1 wt.% to 10 wt.%.

6. The composition of claim 1, wherein the weight ratio of the first EO-PO copolymer component to the second EO-PO copolymer component is 1:1.

7. The composition of claim 1, further comprising a thickener.

8. The composition of claim 1, further comprising an emollient.

9. The composition of claim 8, wherein the emollient is selected from the group consisting of glycerin, sorbitol, lanolin, and mixtures thereof.

10. The composition of claim 7, wherein the thickener is present from 0.1 wt.% to 10 wt.%.

11. The composition of claim 1, wherein the iodine is present from 0.1 wt.% to 2.5 wt.%.

12. The composition of claim 1, wherein the first EO-PO copolymer component is present from 0.1 wt.% to 10 wt.%.

13. The composition of claim 1, wherein the second EO-PO copolymer component is present from 0.1 wt.% to 10.0 wt. %.

## Patentansprüche

1. Wässrige Zitzeneintauchzusammensetzung auf lodbasis, umfassend:
(a) eine EO-PO-Copolymerzusammensetzung, umfassend:
(i) eine erste EO-PO-Copolymerkomponente, die ein zahlendurchschnittliches Molekulargewicht in dem Bereich von 4.500 bis 10.000 aufweist; und
(ii) eine zweite EO-PO-Copolymerkomponente, die ein zahlendurchschnittliches Molekulargewicht in dem Bereich von 1.000 bis 3.600 aufweist;
wobei die erste EO-PO-Copolymerkomponente ein höheres zahlendurchschnittliches Molekulargewicht als das zahlendurchschnittliche Molekulargewicht der zweiten EO-PO-Copolymerkomponente aufweist; und ein Gewichtsverhältnis von der ersten EO-PO-Copolymerkomponente zu der zweiten EO-PO-Copolymerkomponente innerhalb des Bereichs von 2 : 8 bis 8 : 2 liegt;
(b) eine hydrotrope Komponente, die Natriumxylolsulfonat ist; und
(c) eine antimikrobiell wirksame Menge lod;
wobei die Zusammensetzung bei Temperaturen von 4 °C bis 50 °C stabil und im Wesentlichen frei von NPE und LAE ist.

2. Zusammensetzung nach Anspruch 1, wobei die erste EO-PO-Copolymerkomponente ein zahlendurchschnittliches Molekulargewicht von 5.900 bis 6.500 aufweist.

3. Zusammensetzung nach Anspruch 1, wobei die zweite EO-PO-Copolymerkomponente ein zahlendurchschnittliches Molekulargewicht von 1.000 bis 2.900 aufweist.

4. Zusammensetzung nach Anspruch 1, wobei das antimikrobielle Iod aus der Gruppe ausgewählt ist, bestehend aus Iod, Natriumiodid, Kaliumiodid, NaOH/I₂ und Mischungen davon.

5. Zusammensetzung nach Anspruch 1, wobei die Konzentration der hydrotropen Komponente in der Zusammensetzung von 1 Gew.-% bis 10 Gew.-% beträgt.

6. Zusammensetzung nach Anspruch 1, wobei das Gewichtsverhältnis von der ersten EO-PO-Copolymerkomponente zu der zweiten EO-PO-Copolymerkomponente 1 : 1 beträgt.

7. Zusammensetzung nach Anspruch 1, ferner umfassend ein Verdickungsmittel.

8. Zusammensetzung nach Anspruch 1, ferner umfassend einen Weichmacher.

9. Zusammensetzung nach Anspruch 8, wobei der Weichmacher aus der Gruppe ausgewählt ist, bestehend aus Glycerin, Sorbitol, Lanolin und Mischungen davon.

10. Verfahren nach Anspruch 7, wobei das Verdickungsmittel von 0,1 Gew.-% bis 10 Gew.-% vorhanden ist.

11. Verfahren nach Anspruch 1, wobei das lod von 0,1 Gew.-% bis 2,5 Gew.-% vorhanden ist.

12. Verfahren nach Anspruch 1, wobei die erste EO-PO-Copolymerkomponente von 0,1 Gew.-% bis 10 Gew.-% vorhanden ist.

13. Verfahren nach Anspruch 1, wobei die zweite EO-PO-Copolymerkomponente von 0,1 Gew.-% bis 10,0 Gew.-% vorhanden ist.

## Revendications

1. Composition aqueuse à base d'iode pour trempage des trayons, comprenant :
(a) une composition de copolymères EO-PO, comprenant :
(i) un premier composant copolymère EO-PO ayant une masse moléculaire moyenne en nombre dans la plage allant de 4 500 à 10 000 ; et
(ii) un second composant copolymère EO-PO ayant une masse moléculaire moyenne en nombre dans la plage allant de 1 000 à 3 600 ;
le premier composant copolymère EO-PO ayant une masse moléculaire moyenne en nombre plus élevée que la masse moléculaire moyenne en nombre du second composant copolymère EO-PO ; et un rapport massique du premier composant copolymère EO-PO au second composant copolymère EO-PO étant compris dans la plage allant de 2:8 à 8:2 ;
(b) un composant hydrotrope, étant du xylène sulfonate de sodium ; et
(c) une quantité d'iode efficace sur le plan antimicrobien ;
dans laquelle la composition est stable à des températures allant de 4 °C à 50 °C et est sensiblement exempte d'ENP et de LAE.

2. Composition selon la revendication 1, dans laquelle le premier composant copolymère EO-PO a une masse moléculaire moyenne en nombre allant de 5 900 à 6 500.

3. Composition selon la revendication 1, dans laquelle le second composant copolymère EO-PO a une masse moléculaire moyenne en nombre allant de 1 000 à 2 900.

4. Composition selon la revendication 1, dans laquelle l'iode antimicrobien est choisi dans le groupe constitué d'iode, iodure de sodium, iodure de potassium, NaOH/I₂ et mélanges de ceux-ci.

5. Composition selon la revendication 1, dans laquelle la concentration du composant hydrotrope dans la composition va de 1 % en poids à 10 % en poids.

6. Composition selon la revendication 1, dans laquelle le rapport massique du premier composant copolymère EO-PO au second composant copolymère EO-PO est de 1:1.

7. Composition selon la revendication 1, comprenant en outre un épaississant.

8. Composition selon la revendication 1, comprenant en outre un émollient.

9. Composition selon la revendication 8, dans laquelle l'émollient est choisi dans le groupe constitué de glycérine, sorbitol, lanoline et mélanges de ceux-ci.

10. Composition selon la revendication 7, dans laquelle l'épaississant est présent à raison de 0,1 % en poids à 10 % en poids.

11. Composition selon la revendication 1, dans laquelle l'iode est présent à raison de 0,1 % en poids à 2,5 % en poids.

12. Composition selon la revendication 1, dans laquelle le premier composant copolymère EO-PO est présent à raison de 0,1 % en poids à 10 % en poids.

13. Composition selon la revendication 1, dans laquelle le second composant copolymère EO-PO est présent à raison de 0,1 % en poids à 10,0 % en poids.
